(19) Europäisches Patentamt
European Patent Office
Office européen des brevets

(11) Veröffentlichungsnummer: **0 006 114**
**B1**

(12) # EUROPÄISCHE PATENTSCHRIFT

(45) Veröffentlichungstag der Patentschrift: **07.04.82**

(21) Anmeldenummer: **79101205.7**

(22) Anmeldetag: **20.04.79**

(51) Int. Cl.³: **C 07 D 513/04,**
A 61 K 31/505
//C07D277/56, (C07D513/04, 277/00, 239/00)

(54) **7-Methoxy-5-oxo-5H-thiazolo(2,3-b)chinazolin-2-carbonsäure und deren pharmazeutisch verwendbare Salze. Verfahren zu deren Herstellung und sie enthaltende Arzneimittel.**

(30) Priorität: **21.04.78 US 898344**

(43) Veröffentlichungstag der Anmeldung:
**09.01.80 Patentblatt 80/1**

(45) Bekanntmachung des Hinweises auf die Patenterteilung:
**07.04.82 Patentblatt 82/14**

(84) Benannte Vertragsstaaten:
**BE CH DE FR GB IT LU NL SE**

(56) Entgegenhaltungen:
**DE - A - 2 557 425**

**CHEMICAL ABSTRACTS, Vol. 52, Nr. 7
10-04-1958
Columbus, Ohio, U.S.A.
G. SINGH: "Thiopegan derivatives XIV", Spalte 5422 cf
CHEMICAL ABSTRACTS 79, Vol. 79, Nr. 23,
10-12-1973
Columbus, Ohio, U.S.A.
H. K. GAKHAR: "Thiopegan derivatives XXXIX", Seite 337, Spalte 2, Zusammenfassung 137084f**

(73) Patentinhaber: **F. HOFFMANN-LA ROCHE & CO. Aktiengesellschaft
CH-4002 Basel (CH)**

(72) Erfinder: **LeMahieu, Ronald Andrew
18 Spruce Road
North Caldwell, N.J. (US)**

(74) Vertreter: **Lederer, Franz, Dr. et al,
Patentanwälte Dr. Lederer Franz Meyer-Roxlau
Reiner F. Lucile-Grahn-Strasse 22
D-8000 München 80 (DE)**

**EP 0 006 114 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

7-Methoxy-5-oxo-5H-thiazolo(2,3-b)chinazolin-2-carbonsäure und deren pharmazeutisch verwendbare Salze. Verfahren zu deren Herstellung und sie enthaltende Arnzneimittel

Die vorliegende Erfindung betrifft die 7-Methoxy-5-oxo-5H-thiazolo[2,3-b]chinazolin-2-carbonsäure, d.h. die Verbindung der Formel

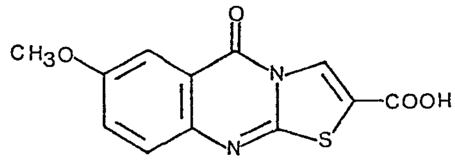
I

sowie pharmazeutisch verwendbare Salze davon.

Der in dieser Beschreibung verwendete Ausdruck "niederes Alkyl" bedeutet Alkylgruppen mit 1—7, vorzugsweise 1—4, Kohlenstoffatomen, wie Methyl, Aethyl, Propyl, Butyl und dergleichen.

7-Methoxy-5-oxo-5H-thiazolo[2,3-b]chinazolin-2-carbonsäure und deren pharmazeutisch verwendbare Salze können erfindungsgemäss so hergestellt werden, dass man

a) ein Anthranilsäurederivat der allgemeinen Formel

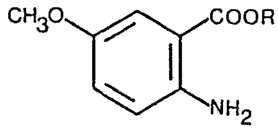
II

worin R Wasserstoff oder niederes Alkyl bedeutet, mit einem Thiazolderivat der allgemeinen Formel

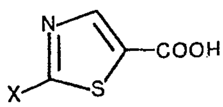
III

worin X Chlor, Brom oder Jod bedeutet, kondensiert, oder

b) 6-Methoxy-2-mercapto-4(3H)-chinazolinon der Formel

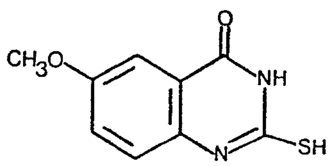
IV

mit einer Verbindung der allgemeinen Formel

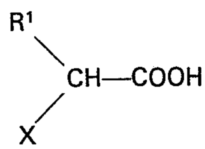
V

worin $R^1$ Formyl bedeutet und X die oben angegebene Bedeutung besitzt, umsetzt, oder

c) eine Verbindung der allgemeinen Formel

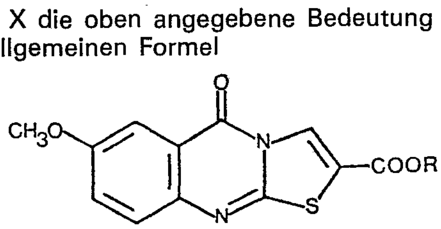
IA

worin R' niederes Alkyl bedeutet, hydrolysiert, und

d) erwünschtenfalls, die erhaltene Verbindung in ein pharmazeutisch verwendbares Salz überführt.

Die Kondensation eines Anthranilsäurederivates der Formel II mit einem Thiazolderivat der Formel III wird vorzugsweise in einem Temperaturbereich von etwa 100 bis etwa 180°C durchgeführt. Die Kondensation kann in Gegenwart oder Abwesenheit eines geeigneten Lösungsmittels durchgeführt werden, gewöhnlicherweise ohne Zugabe eines Lösungsmittels. Zweckmässigerweise kann auch ein hochsiedendes polares Lösungsmittel, wie 2-Methoxyäthanol, Triglyme, Dimethylformamid und dergleichen verwendet werden. Im weiteren kann die Kondensation auch in Gegenwart oder Abwesenheit eines Katalysators durchgeführt werden. Beispiele solcher Katalysatoren sind Alkalimetalljodide, wie Natriumjodid, Lithiumjodid und Kaliumjodid, wobei das Kaliumjodid bevorzugt ist, oder eine geeignete Säure, wie die Ameisensäure und dergleichen.

Die Umsetzung von 6-Methoxy-2-mercapto-4(3H)-chinazolinon mit einer Verbindung der Formel V kann in einem Temperaturbereich von etwa 80 bis etwa 150°C durchgeführt werden. Die Reaktion wird in einem geeigneten Lösungsmittel, wie einem niederen Alkanol, z.B. Isopropanol, n-Butanol oder Aethanol, und dergleichen durchgeführt. Im weiteren kann die Reaktion in Gegenwart oder Abwesenheit eines Katalysators, wie Alkalimetalljodide, z.B. Kaliumjodid, und dergleichen durchgeführt werden.

Die Hydrolyse einer Verbindung der Formel IA zur erwünschten Säure der Formel I kann in einem Ueberschuss eines Alkalimetallhydroxyds, z.B. Natriumhydroxyd, Kaliumhydroxyd und dergleichen, in einem Temperaturbereich von etwa 0 bis etwa 30°C erfolgen. Besonders bevorzugt wird die Reaktion bei etwa 25°C durchgeführt.

Der eigentlichen Hydrolyse folgt ein Erhitzen zum Rückfluss in einer organischen Carbonsäure, wie Essigsäure, Propionsäure und dergleichen. Die erwünschte 7-Methoxy-5-oxo-5H-thiazol[2,3-b]chinazolin-2-carbonsäure kann nach üblichen Verfahrensweisen abgetrennt werden, beispielsweise durch Kristallisation und dergleichen.

Die als Ausgangsmaterial verwendeten Verbindungen der Formeln IA, II, III, IV und V sind bekannt oder können in an sich bekannter Weise, d.h. in zur Herstellung der bekannten Verbindungen analoger Weise, hergestellt werden.

Die Verbindung der Formel I bildet mit Basen pharmazeutisch verwendbare Salze. Beispiele von Basen sind Alkalimetallhydroxyde, wie Natriumhydroxyd, Kaliumhydroxyd und dergleichen, Erdalkalimetallhydroxyde, wie Calciumhydroxyd, Bariumhydroxyd und dergleichen, Alkalimetallalkoholate, wie Natriumäthanolat, Kaliumäthanolat und dergleichen, organische Basen, wie Piperiden, Diäthylamin, N-Methylglucamin und dergleichen.

Die Verbindung der Formel I, d.h. die 7-Methoxy-5-oxo-5H-thiazolo[2,3-b]chinazolin-2-carbonsäure und ihre pharmazeutisch verwendbaren Salze hemmen Hautanaphylaxie bei Ratten und sind daher zur Verhütung von allergischen Reaktionen, z.B. zur prophylaktischen Behandlung von Bronchialasthma, verwendbar. Die anti-anaphylaktische Wirksamkeit kann durch den passiven Hautanaphylaxietest (PCA-Test) bei Ratten gezeigt werden. Bei diesem Test werden Ratten durch Injektion von Antisera in die Haut passiv lokal sensibilisiert. Nach einer Latenzzeit von 24 Stunden wird die Testverbindung, d.h. die 7-Methoxy-5-oxo-5H-thiazolo[2,3-b]chinazolin-2-carbonsäure, intraperitoneal verabreicht. Nach 5 Minuten wird Reagin sowie Evans Blaufarbstoff intravenös appliziert. Die Fälle, die mit einer lokalisierten Antigen-Antikörperreaktion verbunden sind, führen zur Bildung von Hautschwellungen, deren Grösse gemessen wird. Die Fähigkeit der Testverbindung, die Grösse der Hautschwellung die den behandelten Tieren im Vergleich zu den Hautschwellungen der unbehandelten Tiere zu verringern, wird als Mass für die Aktivität der Testverbindungen genommen.

Wird die 7-Methoxy-5-oxo-5H-thiazolo[2,3-b]chinazolin-2-carbonsäure in einer Dosis von 16 mg/kg intraperitoneal verabreicht, beträgt die Verminderung der Grösse der Hautschwellungen 100%.

Wird die 7-Methoxy-5-oxo-5H-thiazolo[2,3-b]chinazolin-2-carbonsäure im obigen Test in einer oralen Dosis von 0,07 mg/kg verabreicht, beträgt die Verminderung der Grösse der Hautschwellungen 50%.

Die 7-Methoxy-5-oxo-5H-thiazolo[2,3-b]chinazolin-2-carbonsäure und ihre pharmazeutisch verwendbaren Salze können oral oder parenteral als anti-allergische Mittel, z.B. für die prophylaktische Behandlung von Bronchialasthma in, den individuellen Erfordernissen angepassten Dosen verabreicht werden. Für therapeutische Zwecke können sie beispielsweise oral oder parenteral durch Verabreichen von therapeutischen Mengen in einer üblichen Darreichungsform, wie Tabletten, Kapseln, Elixiere, Suspensionen, Lösungen, Aerosole und dergleichen verabreicht werden. Sie können in Mischung mit konventionellen pharmazeutischen Trägerstoffen oder Excipientien, wie z.B. Maisstärke, Calciumstearat, Magnesiumcarbonat, Calciumsilikat, Dicalciumphosphat, Talk, Lactose und dergleichen verabreicht werden. Weiterhin können sie in Gegenwart von Puffern oder Stoffen zur Einstellung der Isotonizität verabreicht werden. Die pharmazeutischen Dosierungsformen können erwünschtenfalls konventionellen pharmazeutischen Behandlungsweisen unterworfen werden, beispielsweise können sie sterilisiert werden. Wie bereits oben erwähnt, können die Dosierungen den individuellen Bedürfnissen angepasst werden. Weiterhin können die Darreichungsformen andere therapeutisch wertvolle Substanzen enthalten.

Die Wirkstoffmenge in den einzelnen oben beschriebenen Darreichungsformen ist variabel. Es wird jedoch bevorzugt, Kapseln oder Tabletten zu verabreichen, die von etwa 10 mg bis etwa 20 mg 7-Methoxy-5-oxo-5H-thiazolo[2,3-b]chinazolin-2-carbonsäure oder eine äquivalente Menge eines pharmazeutisch verwendbaren Salzes davon enthalten.

Die Häufigkeit der Verabreichung der Darreichungsform an den Patienten wird in Abhängigkeit von der Wirkstoffmenge in der Darreichungsform und in Abhängigkeit von den Bedürfnissen des Patienten und sonstigen Gegebenheiten erfolgen. Unter gewöhnlichen Umständen können jedoch täglich bis zu etwa 20 mg/kg Wirkstoff in mehreren Dosen verabreicht werden. Es ist darauf hinzuweisen, dass die oben angegebenen Dosierungen lediglich Beispiele darstellen und somit weder den Umfang noch die praktische Anwendung der vorliegenden Erfindung beschränken.

Die nachfolgenden Beispiele erläutern die Erfindung. Alle Temperaturen sind in Celsius-Graden angegeben.

Beispiel 1

5,0 g Methyl 2-aminothiazol-5-carboxylat werden in 54 ml 6N Salzsäure suspendiert und bei —5° bis 0° gerührt. Zu dieser gerührten Suspension werden innerhalb von 15 Minuten tropfenweise 3,7 g Natriumnitrit in 10 ml Wasser getropft. Nach 5-minütigem Rühren wird die erhaltene braune Suspension auf einmal zu einer stark gerührten und auf 5° abgekühlten Suspension von 10,6 g Kupfersulfat und 10,6 g Natriumchlorid gegeben. Das Kühlbad wird entfernt und das Rühren während 30 Minuten fortgesetzt. Man stellt den pH-Wert mit 6N Natriumhydroxydlosung auf 7,3 ein und filtriert die erhaltene grüne Suspension durch Celit. Danach wäscht man den Festkörper mit drei Portionen Aethylacetat und vereinigt diese Extrakte mit dem Aethylacetatextrakt des urpsürnglichen Filtrates. Die vereinigten Extrakte werden über Magnesiumsulfat getrocknet und unter vermindertem Druck zu einem braunen Festkörper eingedampft. Zerreiben mit vier Portionen heissem Petroläther (35—60°) dient dazu, das lösliche Produkt von etwas Ausgangsmaterial abzutrennen. Einengen unter vermindertem Druck der Petrolätherlösung liefert 3,9 g reines Methyl 2-chlorthiazol-5-carboxylat, Schmelzpunkt 41—46°.

Beispiel 2

Eine Mischung von 1,49 g 5-Methoxyanthranilsäure, 1,58 g Methyl 2-chlorthiazol-5-carboxylat und 0,075 g zu Pulver verriebenem Kaliumjodid wird gut gerührt und in einem Oelbad bei 160—165° während 80 Minuten erhitzt. Der erhaltene dunkle Festkörper wird mit 75 ml einer gesättigten Lösung von Natriumbicarbonat behandelt und mit drei Portionen Chloroform extrahiert. Die vereinigten, über Magnesiumsulfat getrockneten Extrakte werden unter vermindertem Druck zu einem braunen Festkörper eingeengt (1,59 g). Kristallisation aus Methylenchlorid/Aetherliefert 0,86 Methyl 7-methoxy-5-oxo-5H-thiazolo[2,3-b]chinazolin-2-carboxylat, Schmelzpunkt 186—192°.

Beispiel 3

0,86 g Methyl 7-methoxy-5-oxo-5H-thiazolo[2,3-b]chinazolin-2-carboxylat wird durch 16-stündiges Rühren bei Raumtemperatur in 30 ml 1N Natriumhydroxydlösung und 30 ml Methanol hydrolysiert. Nach dem Abfiltrieren wird das Filtrat mit Essigsäure auf pH 5 eingestellt und unter vermindertem Druck eingedampft. Dann gibt man Wasser zu und filtriert den Festkörper ab. Da die Basenbehandlung auch den Lactamring öffnet, wird der Festkörper in 75 ml Essigsäure während 1 Stunde zum Rückfluss erhitzt. Nach Einengen auf 25 ml kristallisiert das Produkt beim Abkühlen aus und wird abfiltriert, wobei man 0,53 g reine 7-Methoxy-5-oxo-5H-thiazolo[2,3-b]chinazolin-2-carbonsäure erhält, Schmelzpunkt 247—247,5°.

Beispiel 4

Eine Lösung von 1,000 g (0,006 Mol) 5-Methoxyanthranilsäure und 0,981 g (0,006 Mol) 2-Chlorthiazol-5-carbonsäure in 8 ml 2-Methoxyäthanol, enthaltend 0,08 ml Ameisensäure, wird während 3 1/2 Stunden unter Rückfluss gerührt. Nach dem Abkühlen auf Raumtemperatur gibt man 50 ml Aether zu und filtriert, wobei man 1,083 g eines gelben Festkörpers erhält. Der Festkörper wird mit 25 ml Methanol zerrieben und abfiltriert, wobei man 0,806 g erhält. Umkristallisation aus Essigsäure liefert 0,601 g 7-Methoxy-5-oxo-5H-thiazolo[2,3-b]chinazolin-2-carbonsäure, Schmelzpunkt 241—244°.

Beispiel 5

Eine Lösung von 0,848 g (0,0047 Mol) Methyl 5-methoxyanthranilat und 0,766 g (0,0047 Mol) 2-Chlorthiazol-5-carbonsäure in 7 ml 2-Methoxyäthanol, enthaltend 0,07 ml Ameisensäure, wird während 6 1/2 Stunden unter Rückfluss gerührt. Nach dem Abkühlen auf Raumtemperatur wird der Festkörper abfiltriert. Umkristallisation aus Essigsäure liefert 0,495 g eines gelben Festkörpers. Der Festkörper wird kurz mit überschüssiger, gesättigter Natriumbicarbonatlösung gerührt, das unlösliche Material durch Abfiltrieren entfernt, und das Filtrat abgesäuert. Der erhaltene gelbe Festkörper wird abfiltriert, wobei man 0,260 g 7-Methoxy-5-oxo-5H-thiazolo[2,3-b]chinazolin-2-carbonsäure erhält, Schmelzpunkt 246—249°.

Beispiel 6

Eine Lösung von 0,905 g (0,005 Mol) Methyl 5-methoxyanthranilat und 0,888 g (0,005 Mol) Methyl 2-chlorthiazol-5-carboxylat in 8 ml 2-Methoxyäthanol, enthaltend 0,1 ml Ameisensäure, wird während 2 1/2 Stunden unter Rückfluss gerührt. Nach Abkühlen auf Raumtemperatur werden 10 ml Aether zugegeben, und der gelbe Feststoff abfiltriert. Dieser Festkörper wird mit überschüssiger gesättigter Natriumbicarbonatlösung behandelt, und das Ganze mit Methylenchlorid extrahiert. Der Methylenextrakt wird unter vermindertem Druck zu einem Festkörper eingedampft, der aus Methylenchlorid/Aether umkristallisiert wird, wobei man 0,943 g Methyl 7-methoxy-5-oxo-5H-thiazolo[2,3-b]chinazolin-2-carboxylat erhält, Schmelzpunkt 190—193°.

Beispiel 7

0,943 g Methyl 7-methoxy-5-oxo-5H-thiazolo[2,3-b]chinazolin-2-carboxylat wird durch 17-stündiges Rühren in 3,6 ml 1,0N Natriumhydroxydlösung und 35 ml Methanol hydrolysiert. Man entfernt das Lösungsmittel unter vermindertem Druck, löst den Rückstand in 50 ml Wasser auf und stellt die Lösung mit Salzsäure auf pH 2 ein. Nach dem Abfiltrieren wird der gelbe Feststoff zweimal aus Essigsäure umkristallisiert, wobei man 0,661 g 7-Methoxy-5-oxo-5H-thiazolo[2,3-b]chinazolin-2-carbonsäure erhält, Schmelzpunkt 248—249°.

Beispiel 8

0,044 g Natriummetall (1,92 mMol) wird in 4 ml wasser-freiem Isopropylalkohol gelöst. Dazu gibt man 0,400 g (1,92 mMol) 6-Methoxy-2-mercapto-4(3H)-chinazolinon. Die Reaktionsmischung wird dann während 15 Minuten unter einer Argonatmosphäre zum Rückfluss erhitzt. Nach dem Abkühlen auf Raumtemperatur werden 0,262 g (1,92 mMol) Methylformylchloracetat zugegeben. Die Reaktionsmischung wird dann während 16 Stunden unter Rückfluss gerührt, auf Raumtemperatur abgekühlt, filtriert und mit Wasser gewaschen, wobei man Methyl 7-methoxy-5-oxo-5H-thiazolo[2,3-b]chinazolin-2-carboxylat erhält.

Beispiel A

Kapseln der nachfolgenden Zusammensetzung werden hergestellt:

| | mg/Kapsel | |
|---|---|---|
| | 10 mg | 20 mg |
| 7-methoxy-5-oxo-5H-thiazolo[2,3-b]-chinazolin-2-carbonsäure | 10 | 20 |
| Lactose | 215 | 205 |
| Maisstärke | 60 | 60 |
| Magnesiumstearat | 3 | 3 |
| Talk | 12 | 12 |
| Total | 300 mg | 300 mg |

Verfahren:

Die 7-Methoxy-5-oxo-5H-thiazolo[2,3-b]chinazolin-2-carbonsäure, die Lactose und die Maisstärke werden in einem geeigneten Mischer vermischt, und darauf wird das Ganze durch eine geeignete Mühle gemahlen. Die Mischung wird mit dem Magnesiumstearat und Talk vermischt und auf einer geeigneten Kapselvorrichtung abgefüllt.

Beispiel B

Tabletten der nachfolgenden Zusammensetzung werden hergestellt:

| | mg/Tablette | |
|---|---|---|
| | 10 mg | 20 mg |
| 7-Methoxy-5-oxo-5H-thiazolo-[2,3-b]chinazolin-2-carbonsäure | 10 | 20 |
| Lactose | 182 | 172 |
| mikrokristalline Cellulose | 60 | 60 |
| modifizierte Stärke | 15 | 15 |
| Maisstärke | 30 | 30 |
| Magnesiumstearat | 3 | 3 |
| Total | 300 mg | 300 mg |

Verfahren:

Die ersten fünf Bestandteile werden in einem geeigneten Mischer während 1—15 Minuten vermischt. Man gibt das Magnesiumstearat zu, mischt während 5 Minuten und verpresst auf einer geeigneten Tablettenpresse.

Beispiel C

Tabletten der nachfolgenden Zusammensetzung werden hergestellt:

| | mg/Tablette | |
|---|---|---|
| | 10 mg | 20 mg |
| 7-Methoxy-5-oxo-5H-thiazolo-[2,3-b]chinazolin-2-carbonsäure | 10 | 20 |
| Lactose | 264 | 254 |
| vorgelatinierte Stärke | 17,5 | 17,5 |
| Maisstärke | 35 | 35 |
| modifizierte Stärke | 17,5 | 17,5 |
| Magnesiumstearat | 6 | 6 |
| Total | 350 mg | 350 mg |

Verfahren:

Die ersten vier Bestandteile werden in einem geeigneten Mischer vermischt und mit Wasser granuliert. Das Ganze wird danach durch eine geeignete Mühle gemahlen. Dann mischt man die modifizierte Stärke und das Magnesiumstearat zu und verpresst auf einer geeigneten Presse zu Tabletten.

**Patentansprüche**

1. 7-Methoxy-5-oxo-5H-thiazolo[2,3-b]chinazolin-2-carbonsäure und pharmazeutisch verwendbare Salze davon.

2. 7-Methoxy-5-oxo-5H-thiazolo[2,3-b]chinazolin-2-carbonsäure und pharmazeutisch verwendbare Salze davon als pharmazeutische Wirkstoffe.

3. 7-Methoxy-5-oxo-5H-thiazolo[2,3-b]chinazolin-2-carbonsäure und pharmazeutisch verwendbare Salze davon als Antiallergika.

4. Verfahren zur Herstellung von 7-Methoxy-5-oxo-5H-thiazolo[2,3-b]chinazolin-2-carbonsäure und den pharmazeutisch verwendbaren Salzen davon, dadurch gekennzeichnet, dass man

a) ein Anthranilsäurederivat der allgemeinen Formel

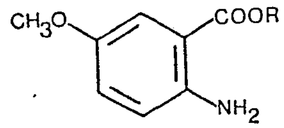

II

worin R Wasserstoff oder niederes Alkyl bedeutet, mit einem Thiazolderivat der allgemeinen Formel

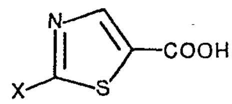

III

worin X Chlor, Brom oder Jod bedeutet, kondensiert, oder

b) 6-Methoxy-2-mercapto-4(3H)-chinazolinon der Formel

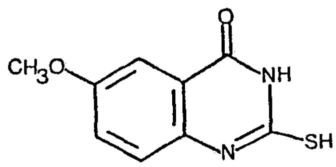

IV

mit einer Verbindung der allgemeinen Formel

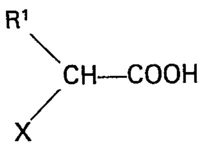

V

worin $R^1$ Formyl bedeutet und X die oben angegebene Bedeutung bezitzt, umsetzt, oder

c) eine Verbindung der allgemeinen Formel

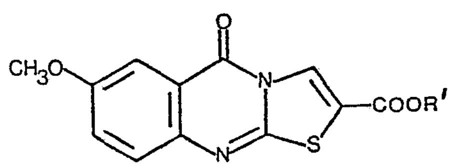

IA

worin R' niederes Alkyl bedeutet, hydrolysiert, und

d) erwünschtenfalls, die erhaltene Verbindung in ein pharmazeutische verwendbares Salz überführt.

5. Arzneimittel, enthaltend 7-Methoxy-5-oxo-5H-thiazolo[2,3-b]chinazolin-2-carbonsäure oder ein pharmazeutisch verwendbares Salz davon.

6. Antiallergikum, enthaltend 7-Methoxy-5-oxo-5H-thiazolo[2,3-b]chinazolin-2-carbonsäure oder ein pharmazeutisch verwendbares Salz davon.

## Claims

1. 7-Methoxy-5-oxo-5H-thiazolo[2,3-b]quinazoline-2-carboxylic acid and pharmaceutically useable salts thereof.

2. 7-Methoxy-5-oxo-5H-thiazolo[2,3-b]quinazoline-2-carboxylic acid and pharmaceutically useable salts thereof as pharmaceutically active substances.

3. 7-Methoxy-5-oxo-5H-thiazolo[2,3-b]quinazoline-2-carboxylic acid and pharmaceutically useable salts thereof as antiallergics.

4. Process for the manufacture of 7-methoxy-5-oxo-5H-thiazolo[2,3-b]quinazoline-2-carboxylic acid and the pharmaceutically useable salts thereof, characterised by

a) condensing an anthranilic acid derivative of the general formula

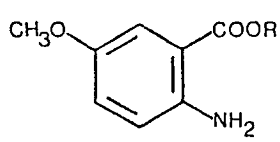

II

wherein

R signifies hydrogen or lower alkyl, with a thiazole derivative of the general formula

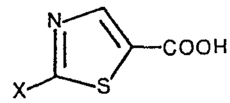

III

wherein

X signifies chlorine, bromine or iodine, or

b) reacting 6-methoxy-2-mercapto-4(3H)-quinazolinone of the formula

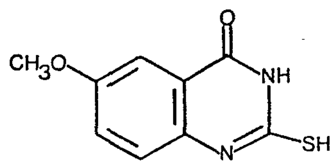

IV

with a compound of the general formula

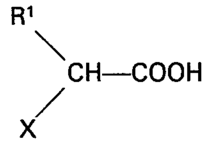

V

wherein

R' signifies formyl and X has the significance given above, or

c) hydrolysing a compound of the general formula

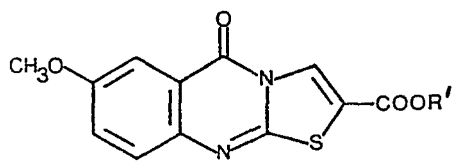

IA

wherein

R' signifies lower alkyl, and

d) if desired, converting the compound obtained into a pharmaceutically useable salt.

5. Medicament, containing 7-methoxy-5-oxo-5H-thiazolo[2,3-b]quinazoline-2-carboxylic acid or a pharmaceutically useable salt thereof.

6. Antiallergic, containing 7-methoxy-5-oxo-5H-thiazolo[2,3-b]quinazoline-2-carboxylic acid or a pharmaceutically useable salt thereof.

**Revendications**

1. L'acide 7-méthoxy-5-oxo-5H-thiazolo[2,3-b]quinazoline-2-carboxylique et ses sels pharmaceutiquement acceptables.

2. L'acide 7-méthoxy-5-oxo-5H-thiazolo[2,3-b]quinazoline-2-carboxylique et ses sels pharmaceutiquement acceptables en tant que substances actives pharmaceutiques.

3. L'acide 7-méthoxy-5-oxo-5H-thiazolo[2,3-b]quinazoline-2-carboxylique et ses sels pharmaceutiquement acceptables en tant qu'agents antiallergiques.

4. Procédé de préparation de l'acide 7-méthoxy-5-oxo-5H-thiazolo[2,3-b]quinazoline-2-carboxylique et de ses sels pharmaceutiquement acceptables, caractérisé en ce que

a) on condense un dérivé de l'acide anthranilique répondant à la formule générale

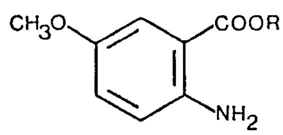

II

dans laquelle R représente l'hydrogène ou un groupe alkyle inférieur, avec un dérivé de thiazole répondant à la formule générale

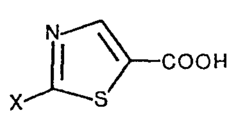

III

dans laquelle X représente le chlore, le brome ou l'iode, ou

b) on traite la 6-méthoxy-2-mercapto-4(3H)-quinazolinone répondant à la formule

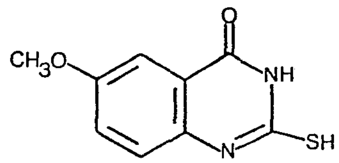

IV

par un composé répondant à la formule générale

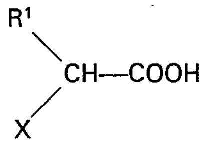

V

dans laquelle $R^1$ représente le groupe formyle et X a les significations indiquées ci-dessus, ou

c) on hydrolyse un composé répondant à la formule générale

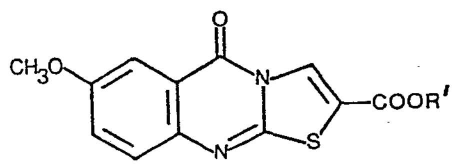

IA

dans laquelle R′ représente un groupe alkyle inférieur, et

d) si on le désire, on convertit le composé obtenu en un sel pharmaceutiquement acceptable.

5. Médicament contenant de l'acide 7-méthoxy-5-oxo-5H-thiazolo[2,3-b]quinazoline-2-carboxylique ou l'un de ses sels pharmaceutiquement acceptables.

6. Agent antiallergique contenant de l'acide 7-méthoxy-5-oxo-5H-thiazolo[2,3-b]quinazoline-2-carboxylique ou l'un de ses sels pharmaceutiquement acceptables.